# EUROPÄISCHE PATENTSCHRIFT

(11) **EP 0 716 808 B1**
(45) Veröffentlichungstag und Bekanntmachung des Hinweises auf die Patenterteilung: **19.09.2001**
(21) Anmeldenummer: 95119190.7
(22) Anmeldetag: 06.12.1995
(51) Int. Cl.: A01N 37/10, A01N 43/38, A01N 43/40, A01N 43/42, A01N 37/38

(54) **Verwendung von Herbiziden vom Auxin-Typ zur Behandlung von transgenen Kulturpflanzen**
Use of Auxin-type herbicides for the treatment of transgenic crops
Utilisation des herbicides du type Auxin pour le traitement des récoltes

(30) Priorität: 15.12.1994 DE 4444708
(43) Veröffentlichungstag der Anmeldung: 19.06.1996
(73) Patentinhaber: BASF AKTIENGESELLSCHAFT, 67056 Ludwigshafen (DE)
(72) Erfinder: Grossman, Klaus, Prof. Dr., D-67117 Limburgerhof (DE); Walter, Helmut Dr., D-67283 Obrigheim (DE)

(56) Entgegenhaltungen:
- WO-A-91/09112
- WO-A-92/04456
- WO-A-95/16047
- PLANT GROWTH REGUL., Bd. 16, 1995, Seiten 183-188, XP002023294 K. GROSSMANN & T. SCHMÜLLING: "The effects of the herbicide quinclorac on shoot growth in tomato is alleviated by inhibitors of ethylene biosynthesis and by the presence of an antisense construct of the ACC synthase gene in transgenic plants."
- J. PLANT PHYSIOL., Bd. 142, 1993, Seiten 457-466, XP002023295 K. GROSSMANN & J. KWIATKOWSKI: "Selective induction of ethylene and cyanide biosynthesis appears to be involved in the selectivity of the herbicide Quinclorac between rice and barnyardgrass."
- EUPHYTICA, Bd. 79, 1994, Seiten 251-263, XP002023296 D. GRIERSON & R. FRAY: "Control of ripening in transgenic tomatoes"
- S. J. KOO, J. C. NEAL & J. M. DITOMASO: "3,7-Dichloroquinolinecarboxylic acid inhibits cell-wall biosynthesis in maize roots.", PLANT PHYSIOL., , , Band 112, Nr. , Seiten 1383 - 1389
- K. GROSSMANN: "A role for cyanide, derived from ethylene biosynthesis, in the development of stress symptoms.", PHYSIOLOGIA PLANTARUM, , , Band 97, Nr. , Seiten 772 - 775
- S. ABEL & A. THEOLOGIS: "Early genes and auxin action.", PLANT PHYSIOL., , , Band 111, Nr. , Seiten 9 - 17
- K. GROSSMANN & F. SCHELTRUP: "Studies on the mechanism of selectivity of the auxin herbicide Quinmerac.", PESTIC. SCI., , , Band 52, Nr. , Seiten 111 - 118

## Beschreibung

Die vorliegende Erfindung betrifft die Verwendung von Chinolincarbonsäure-Herbiziden vom Auxin-Typ zur Bekämpfung von Unkräutern und Schadgräsern in transgenen Tomatenpflanzen, die ein ACC Synthase Antisense Gen, ACC Oxidase Antisense Gen, ACC Deaminase Gen oder deren Kombinationen enthalten.

Herbizide vom Auxin-Typ interferieren mit pflanzlichen Wuchsstoffen, den Auxinen, wobei sie Metabolisierungsprozesse stimulieren, zu abartiger Morphologie und schließlich zu einem Zerfall des Gewebes und Absterben der Pflanzen führen. Mit der Wirkung der Herbizide vom Auxin-Typ ist die Stimulierung der Biosynthese des Ethylens verbunden (Target sites for herbizide action, Plenum Press, New York, 1991, Seite 155, Morgan, P.W. (1976) Effect of ethylene physiology. In: Herbicides physiology, biochemistry, and ecology, Seite 256-280 (Audus, L.J., ed.) Academic Press, New York). Die Herbizide vom Auxin-Typ zeigen exzellente Wirkung gegen ein breites Spektrum von Unkräutern und Schadgräsern. Grossmann und Kwiatkowski, J. Plant Physiol. 142 (1993), 457-466 beschreiben, daß das Chinolincarbonsäurederivat Quinclorac selektiv in Gräsern wirkt. Es schädigt bei entsprechender Dosierung Schadgräser wie Echinochloa crus-galli, wobei die Kulturpflanze Reis gegenüber dem Herbizid tolerant ist. In WO 91/09112 wird auf die Verringerung der Ethylenbildung nach Zugabe des synthetischen Auxins NAA in AdoMetase-transformiertem Tabakgewebe hingewiesen. WO 95/16047 beschreibt eine Methode zur Selektion Ethylen-resistenter Pflanzen. Obwohl einige Kulturpflanzen wie z.B. Reis, Raps und Weizen tolerant gegenüber-beispielsweise Quinclorac sind, werden andere Kulturpflanzen wie Tomaten, Soja, Baumwolle und Mais von Herbiziden vom Auxin-Typ angegriffen.

Herbizide vom Auxin-Typ sind bekannt und werden beispielsweise in der DE-OS 31 08 873 und DE-OS 32 33 089 beschrieben.

Aus der WO 92/04456 sind transgene Pflanzen bekannt, die ein ACC Synthase Antisense Gen enthalten. Dieses Gen inhibiert die Synthese von 1-Aminocyclopropan-1-carbonsäure (ACC) in der Ethylenbiosynthese, wodurch z.B. in Tomaten die Reifung unterdrückt wird, was sich daran zeigt, daß die Tomaten über Monate grün bleiben.

Aufgabe der vorliegenden Erfindung war es, einen Weg aufzuzeigen, der es ermöglicht, Herbizide vom Auxin-Typ auch zur Bekämpfung von Unkräutern und Schadgräsern in Kulturpflanzen zu verwenden, die normalerweise nicht tolerant gegenüber diesen Herbiziden sind.

Überraschenderweise wurde gefunden, daß diese Aufgabe gelöst werden konnte durch die Verwendung von Chinolincarbonsäure-Herbiziden vom Auxin-Typ zur Bekämpfung von Unkräutern und Schadgräsern in transgenen Tomatenpflanzen, die ein ACC Synthase Antisense Gen, ACC Oxidase Antisense Gen, ACC Deaminase Gen oder deren Kombinationen enthalten.

Herbizide vom Auxin-Typ sind bekannt und werden in Ralph C. Kirkwood, Target sites for herbizide action, 1991 Plenum Press, New York, Seite 154-167 (Autor K.E. Pallett); Carl Fedtke, Biochemistry and physiology of herbicide action, Springer Verlag Berlin, Heidelberg 1982, Seiten 159-176; DE-OS 31 08 873 und DE-OS 32 33 089 beschrieben.

Beschrieben werden beispielsweise Herbizide vom Auxin-Typ wie Phenoxyessigsäuren, Benzoesäuren, Pyridincarbonsäuren, Chinolincarbonsäuren, durch Aromaten oder Heteroaromaten substituierte Essigsäuren sowie einige Spezialtypen.

Geeignete Phenoxycarbonsäuren sind 2,4-Dichlorphenoxyessigsäure (2,4-D), 2,4-Dichlorphenoxybuttersäure (2,4-DB), 2,4-Dichlorphenoxypropionsäure (2,4-DP, Dichlorprop), 4-Chlor-2-methylphenyoxyessigsäure (MCPA), (+)-2-(4-Chlor-2-methylphenoxy)propionsäure (Mecoprop), 2,4,5-Trichlorphenoxyessigsäure (2,4,5-T), 3,5,6-Trichlor-2-pyridyloxyessigsäure (Triclopyr) sowie deren Salze, Ester und Amide.

Geeignete Benzoesäuren sind beispielsweise 3-Amino-2,5-dichlorbenzoesäure (Chloramben), 3,6-Dichlor-2-methoxybenzoesäure (Dicamba), 2,3,6-Trichlorbenzoesäure (2,3,6-TBA), 3,5,6-Trichlor-2-methoxybenzoesäure (Tricamba) sowie deren Salze, Ester und Amide.

Geeignete Pyridincarbonsäuren sind beispielsweise 3,6-Dichlorpyridin-2-carbonsäure (Clopyralid), 4-Amino-3,5,6-trichloropyridin-2-carbonsäure (Picloram) sowie deren Salze, Ester und Amide.

Geeignete Chinolincarbonsäuren sind beispielsweise 3,7-Dichlorchinolin-8-carbonsäure (Quinclorac), 7-Chlor-3-methylchinolin-8-carbonsäure (Quinmerac) sowie deren Salze, Ester und Amide.

Geeignete durch Aromaten oder Heteroaromaten substituierte Essigsäuren sind beispielsweise 4-Chlor-2,3-dihydro-2-oxo-1,3-benzothiazol-3-ylessigsäure (Benazolin), 2,3,6-Trichlorphenylessigsäure (Fenac), Indol-3-ylessigsäure (IAA), 1-Naphthylessigsäure (NAA) sowie deren Salze, Ester und Amide.

Einige Spezialtypen von Herbiziden vom Auxin-Typ sind beispielsweise Orthonil und 4-Amino-3,5-dichlor-6-fluor-2-pyridyl-oxyessigsäure (Fluoroxypyr).

Bevorzugt werden Quinclorac und Quinmerac verwendet.

Transgene Kulturpflanzen, die ein l-Aminocyclopropan-1-carbonsäure (ACC) Synthase Antisense Gen enthalten, sind in der WO 92/04456 beschrieben, einschließlich deren gentechnische Herstellung sowie die Isolation der zugrundeliegenden DNS. Dieses ACC Synthase Antisense Gen ist verantwortlich dafür, daß die Synthese der direkten Ethylenvorstufe l-Aminocyclopropan-1-carbonsäure (ACC) in der Biosynthese des Phytohormons Ethylen verhindert wird, wodurch der Reifeprozeß der Kulturpflanze gestoppt wird, was sich z.B. bei Tomaten am Ausbleiben der Rotfärbung zeigt.

Nach J.E. Gray, Plant, Cell and Environment (1994) 17, 557 bis 571, greift neben der ACC Synthase auch die ACC Oxidase und die ACC Deaminase in die pflanzliche Ethylenbiosynthese ein. Die ACC Synthase wie auch die ACC Oxidase werden in der Pflanze durch Genfamilien kodiert. Die Expression dieser Gene wird abhängig von der Pflanzenentwicklung bzw. durch Einflußnahme von Umweltfaktoren induziert. ACC Deaminase Gene wurden bisher aus Bodenbakterien isoliert und in transgene Pflanzen eingeschleust. Während die ACC Oxidase Antisense Gene die Umwandlung von ACC in Ethylen inhibieren, führen ACC Deaminase Gene, in Sense Orientierung in die Pflanze eingebracht, zum Abbau des synthetisierten ACC. In allen Fällen wird die Bildung des Phytohormons Ethylen dadurch reduziert oder fast vollständig gehemmt.

Es wurde nun überraschend gefunden, daß die in der WO 92/04456 beschriebenen transgenen Kulturpflanzen, die ein ACC Synthase Antisense Gen enthalten, gegenüber den oben genannten Chinolincarbonsäure-Herbiziden vom Auxin-Typ tolerant sind.

Die Chinolincarbonsäure-Herbizide vom Auxin-Typ bzw. die sie enthaltenden herbiziden Mittel sowie deren umweltverträgliche Salze, z.B. von Alkalimetallen, Erdalkalimetallen oder Ammoniak und Aminen bzw. die sie so enthaltenden herbiziden Mittel, können in den transgenen Tomatenkulturen, Unkräuter und Schadgräser sehr gut bekämpfen, ohne die transgenen Kulturpflanzen zu schädigen, ein Effekt, der vor allem auch bei niedrigen Aufwandmengen auftritt.

Die Applikation der herbiziden Mittel bzw. der Chinolincarbonsäure-Herbizide vom Auxin-Typ kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Chinolincarbonsäure-Herbizide vom Auxin-Typ bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryl- ether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 bis 100 %, vorzugsweise 95 bis 100 % (nach NMR-Spektrum) eingesetzt.

Die Chinolincarbonsäure-Herbizide vom Auxin-Typ können beispielsweise wie folgt formuliert werden:
I. 20 Gewichtsteile 3,7-Dichlorchinolin-8-carbonsäure (Quinclorac) werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.% des Wirkstoffs enthält.
II. 20 Gewichtsteile 7-Chlor-3-methylchinolin-8-carbonsäure (Quinmerac) werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile 3,7-Dichlorchinolin-8-carbonsäure werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.% des Wirkstoffs enthält.
IV. 20 Gewichtsteile 3,7-Dichlorchinolin-8-carbonsäure werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.% des Wirkstoffs enthält.
V. 3 Gewichtsteile 3,7-Dichlorchinolin-8-carbonsäure werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile 3,7-Dichlorchinolin-8-carbonsäure werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Zur Verbreiterung des Wirkungsspektrums und zur Erzielung synergistischer Effekte können die Chinolincarbonsäure-Herbizide vom Auxin-Typ mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dion-derivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Chinolincarbonsäure-Herbizide vom Auxin-Typ allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0 kg/ha, vorzugsweise 0,01 bis 1,0 kg/ha aktive Substanz (a.S.).

### Beispiel

Wild-Typ Tomatenpflanzen (Sorte VF-36, nicht transformiert) und homozygote Antisense Pflanzen des Transformanten A 11.1 (A 11.1 - 10 - 17; fünfte Generation), die das "constitutively expressed antisense construct pPO 35 to ACC Synthase Gene LE-ACC 2" (Oeller PW, Min-Wong L, Taylor LP, Pike DA and Theologis A (1991) Reversible inhibition of tomato fruit senescence by antisense RNA, Science 254; 437 bis 439) enthielt, wurden in Erde in 200-ml-Töpfen (eine Pflanze pro Topf) bei einem Licht/Dunkel-Wechsel von 15/9 Stunden bei 24/20°C kultiviert. Das Saatgut wurde vom Plant Gene Expression Center, Albany, CA, U.S.A., zur Verfügung gestellt. Im zweiten Blattstadium, 3 Wochen nach der Aussaat, wurden die Pflanzen (5 Wiederholungen) über den Boden mit einer einmaligen Anwendung von 20 ml pro Pflanze einer wäßrigen Lösung von 0,1, 0,5 und 5 mg Quinclorac behandelt. Dabei wurde der Wirkstoff in 0,4 ml eines Gemisches aus 1 Volumenanteil DMSO und 10 Volumenanteilen Methanol gelöst und mit Wasser verdünnt.

Den unbehandelten Kontrollpflanzen wurde nur das Lösungsmittel appliziert. Die Wachstumsparameter wurden 12 Tage nach der Behandlung bestimmt. Tabelle 1 zeigt den Einfluß von Quinclorac auf Sproßfrischmasse und Pflanzenhöhe der Wild-Typ Pflanze (VF-36). Tabelle II zeigt den Einfluß des Quinclorac auf Sproßfrischmasse und Pflanzenhöhe der Antisense Pflanze (A 11.1). Die Daten sind jeweils Mittelwerte von je 5 Tomatenpflanzen (± Standardfehler). Werte mit gleichen Buchstaben sind nicht signifikant verschieden (p = 0,1, Duncan's multiple range test).

**Tabelle I**

| Wild-Typ Tomate (VF-36) | | |
|---|---|---|
| Quinclorac [mg/Pflanze] | Sproßfrischmasse [g] | Pflanzenhöhe [cm] |
| 0 | 5,8 ± 0,5 a | 16,1 ± 1,0 a |
| 0,1 | 3,9 ± 0,7 b | 12,1 ± 0,8 b |
| 0,5 | 2,6 ± 0,4 c | 11,6 ± 0,6 b |
| 5,0 | 0,9 ± 0,2 d | 8,5 ± 0,9 c |

**Tabelle II**

| Antisense Tomate (A 11.1) | | |
|---|---|---|
| Quinclorac [mg/Pflanze] | Sproßfrischmasse [g] | Pflanzenhöhe [cm] |
| 0 | 5,8 ± 0,5 a | 15,4 ± 0,6 a |
| 0,1 | 6,7 ± 0,9 a | 16,1 ± 1,3 a |
| 0,5 | 5,1 ± 0,8 a | 14,7 ± 0,8 a |
| 5,0 | 0,8 ± 0,2 b | 8,2 ± 0,4 b |

Die Werte der Tabelle II zeigen eindeutig die Toleranz der Antisense Tomate gegenüber Quinclorac bis zu einer Menge von 0,5 mg/Pflanze. Erst bei 5 mg wird die Tomatenpflanze geschädigt, wohingegen die Schädigung der Wild-Typ Tomatenpflanze schon bei 0,1 mg/Pflanze Quinclorac beginnt.

## Patentansprüche

1. Verwendung von Chinolincarbonsäure-Herbiziden vom Auxin-Typ zur Bekämpfung von Unkräutern und Schadgräsern in transgenen Tomatenpflanzen, die ein ACC Synthase Antisense Gen, ACC Oxidase Antisense Gen, ACC Deaminase Gen oder deren Kombinationen enthalten.

2. Verwendung von Chinolincarbonsäure-Herbiziden vom Auxin-Typ, sowie deren Salze, Ester und Amide, gemäß Anspruch 1.

3. Verwendung von Chinolincarbonsäure-Herbiziden vom Auxin-Typ gemäß einem der Ansprüche 1 bis 2, wobei Quinmerac oder Quinclorac eingesetzt wird.

4. Verwendung von Herbiziden vom Auxin-Typ gemäß einem der Ansprüche 1 bis 3, wobei transgene Tomatenpflanzen ausgewählt werden, die ein ACC Synthase Antisense Gen enthalten.

## Claims

1. The use of quinolinecarboxylic acid herbicides of the auxin type for controlling broad-leaved weeds and grass weeds in transgenic tomato plants which contain an ACC synthase antisense gene, ACC oxidase antisense gene, ACC deaminase gene or combinations thereof.

2. The use of quinolinecarboxylic acid herbicides of the auxin type and their salts, esters and amides, as claimed in claim 1.

3. The use of quinolinecarboxylic acid herbicides of the auxin type as claimed in either of claims 1 or 2, quinmerac or quinclorac being employed.

4. The use of herbicides of the auxin type as claimed in one of claims 1 to 3, transgenic tomato plants being selected which contain an ACC synthase antisense gene.

## Revendications

1. Utilisation d'herbicides de type auxine à base d'acide quinoléine-carboxylique pour la lutte contre les mauvaises herbes et les graminées nuisibles dans des plants de tomates transgéniques, qui contiennent un gène antisens de synthase d'ACC, un gène antisens d'oxydase d'ACC, un gène de désaminase d'ACC ou leurs combinaisons.

2. Utilisation d'herbicides de type auxine à base d'acide quinoléine-carboxylique, ainsi que de ses sels, esters et amides, selon la revendication 1.

3. Utilisation d'herbicides de type auxine à base d'acide quinoléine-carboxylique selon l'une des revendications 1 à 2, dans laquelle du Quinmerac ou du Quinclorac est employé.

4. Utilisation d'herbicides de type auxine selon l'une des revendications 1 à 3, dans laquelle on choisit des tomates transgéniques qui contiennent un gène antisens de synthase d'ACC.
